# EUROPEAN PATENT APPLICATION

(11) **EP 1 512 399 A1**
(43) Date of publication of application: **09.03.2005**
(21) Application number: 03733267.3
(22) Date of filing: 03.06.2003
(51) Int. Cl.: A61K 31/409, A61K 9/107, A61K 47/48, A61K 41/00, A61P 35/00, A61P 43/00

(54) **PORPHYRIN OXYGEN INFUSION PREPARATION FOR INCREASING OXYGEN CONCENTRATION IN TUMOR TISSUE**

(30) Priority: 03.06.2002 JP 2002161942
(71) Applicant: Nipro Corporation, Osaka-shi, Osaka 531-8510 (JP)
(72) Inventor: TSUCHIDA, Eishun, Nerima-ku, Tokyo 177-0053 (JP); KOBAYASHI, Koichi, Musashino-shi, Tokyo 180-0022 (JP); KOMATSU, Teruyuki, Tachikawa-shi, Tokyo 190-0023 (JP); HORINOUCHI, Hirohisa, Meguro-ku, Tokyo 153-0051 (JP); WATANABE, Masumi, Taito-ku, Tokyo 111-0052 (JP)
(74) Representative: Hayes, Adrian Chetwynd
(86) International application number: PCT/JP2003/006991
(87) International publication number: WO 2003/101451

(57) **Abstract**

A highly safe oxygen infusion for effectively increasing an oxygen partial pressure in a hypoxic region of tumor tissues, which comprises a dispersion of an albumin clathrate compound enclosing a porphyrin metal complex, dispersed in a physiologically permissible aqueous media.

## Description

### Technical Field

The present invention relates to a porphyrin oxygen infusion for increasing oxygen concentrations in tumor tissues, which is administered to mammals having tumor tissues to increase oxygen partial pressures in a hypoxic region of the tumor tissues.

### Background Art

In living bodies, hemoglobins in red blood cells are responsible for oxygen transport. There have been reported many researches on reproduction of an oxygen-transport function similar to that of an iron(II) protoporphyrin complex that is an oxygen-binding pocket for hemoglobin, by use of various synthetic compounds. For example, pioneering reports include J.P. Collman, Acc. Chem. Res., 10, 265 (1977), and F. Basolo, B. M. Hoffman, J. A. Ibers, Ibid, 8, 384 (1975). In particular, known as an iron (II) Porphyrin complex that can form a stable oxygen complex under room temperature is 5,10,15,20-tetrakis (α,α,α,α-o-pival-amidophenyl) porphyrin iron(II) complex (hereinafter referred to as a "FeTpivPP complex") (J. P. Collman, et al., J. Am. Chem. Soc., 97, 1427 (1975). The FeTpivPP complex can reversibly bind or release molecular oxygen at room temperature in the presence of an axial base (such as 1-alkylimidazole, 1-alkyl-2-methylimidazole, or a derivative of pyridine) in an organic solvent such as benzene, toluene, dichloromethane, N, N-dimethylformamide, tetrahydrofuran or the like. However, when the FeTpivPP complex is intended to be used in the living body as an artificial oxygen carrier (an oxygen infusion) that can exhibit the oxygen transport function in stead of hemoglobin, it is essential for the FeTpivPP complex to have an ability to bind or release oxygen under the physiological conditions (i.e., at pH 7.4, Temperature < 40 °C in physiological saline). The present inventors have succeeded in realizing oxygen infusions that can reversibly bind and release oxygen even under the physiological conditions by making active use of minute hydrophobic environments constructed in the vicinity of oxygen coordinating sites along with solubilization of the FeTpivPP complex in water, which are achieved by various methods such as, for example, a method for embedding the FeTpivPP complex or their analogues in bilayer membrane endoplasmic reticula comprising phospholipids (Dalton Trans., 1984, 1147, JP S58-21371(A); a method for enclosing or covering the FeTpivPP complex with micro spheres comprising guttate oil globules (E. Tsuchida et al., Biochem. Biophs, Acta., 1108, 253-256 (1992), JP H06-264641(A)); a method of self-assembly by inducing formation of covalent bonds with amphipathic substituents (JP H06-92966(A)); and a method for enclosing serum albumin in hydrophobic domains (JP H08-301873(A)). Further, the inventors have proved that these oxygen infusions have an ability to sufficiently transport oxygen even when administered to the living body (E. Tsuchida et. al., Artif. Organs Today, 5, 207-215 (1996)).

As mentioned above, in order to allow the FeTpivPP complex to exert reversible oxygen binding and releasing properties, it is necessary to externally add a basic axial ligand in an excess number of moles to the liquid. The present inventors have realized a system that can produce stable oxygen complex without addition of any basic axial ligand, by incorporating, for example, an alkyl imidazole derivative or an alkyl histidine derivative as a substituent into molecules of the iron(II) porphyrin complex to form a covalent bond therewith (JP H05-85141(A)). Some of the imidazole derivatives, which have been widely used as an axial base, have medicinal properties, but they are mostly highly toxic to the body tissues. Further, if the used carrier is phospholipid endoplasmic reticulum, ripido microspheres or albumin, the excessively coexisted imidazole derivative may be a factor contributing to destabilization of the morphologic feature. As a way to minimize the added amount of the axial base, the inventors had no choice but to incorporate the imidazole derivative into molecules of the iron(II) porphyrin complexes. Of course, it has been continuously and experimentally proved that the resultant modified iron(II) porphyrin complexes function as oxygen carriers that can be administered to the living bodies (E. Tsuchida et al., Bioconjugate Chem., 11, 46-50 (2000)).

The oxygen infusions have extremely wide applications in medical cares. The expected applications include not only use as revival liquids (alternative red blood cells) for hemorrhagic shock, but also use as oxygen carriers for transporting oxygen to ischemic sites in myocardial infarction, perfusion or stock solutions for transplantable organs, compensating solutions for extracorporeal circulation circuits such as artificial hearts and lungs, oxygen carriers for transporting oxygen to cultured cells of regenerating tissues. Further, recently there is an increasing interest in application of the oxygen infusion as a cancer therapy intensifier, i.e., its curative properties against hypoxic region in tumor tissues.

In general, the cancer cells are hypoxic cells, and the presence of the hypoxic cells is one of the reasons that malignant tumors have resistance to radiotherapy or chemotherapy. The hypoxic cells include (i) acute hypoxic cells caused by the fact that a blood flow in a tumor site is temporarily changed, which in turn causes suspension of oxygen to be transported to regions subject to a certain blood vessel, and (ii) chronic hypoxic cells derived from the fact that formation of new blood vessels can not keep up with abnormal growth of tumors, causing insufficient oxygen supply to cells away from the blood cells. In fact, under the presence of oxygen, the radio sensitivity of the tumor tissues is enhanced up to 3 times compared to that observed under oxygen-free condition, and the chances of survival of the tumor tissues are reduced. The radiosensitizing effect is remarkably observed at an oxygen concentration of 0 Torr to 40 Torr, but almost unchanged at a concentration exceeding that range.

There are many unclear points in the action mechanism of oxygen effects. For example, molecular oxygen is a strong oxidant that possesses a high electron affinity. However, the radiosensitizing effect due to oxygen is not increased in a simple aqueous solution, and the oxygen effect is never induced even when DNA molecules that are considered as a target substance are exposed to radiation in its aqueous solution. At present, it is believed that the oxygen effect inside the cells is caused by antagonism between oxygen and glutathione (GSH). The reason why the cells are killed has been believed that intracellular target molecules (DNA) form radicals in the cells by the direct or indirect action of radiation. The radicals are decreased by reducing reaction of GSH contained in the cells, and repair radiation damage of the cells. In that case, however, if oxygen exists in large quantity, oxygen blocks the action of GSH to produce oxygen effects.

Up to now, there are some reports on attempts to improve anticancer properties and radio sensitivity by administration of an oxygen infusion to increase the oxygen concentration of the tumor tissue in low-oxygen conditions. The attention was paid to utility of a perfluorochemical (PFC) emulsion as an oxygen infusion. In 1982, Kokuuchi et al. reported for the first time a combined therapy of the PFC emulsion and chemical therapy. They studied changes of an oxygen concentration in brain tumor tissues caused by administration of PFC emulsion, using rat models of subcutaneously transplanted brain tumors (Kokuuchi et al. Cancer and chemical therapy, 11, 2207-2211 (1984). The administration of the PFC emulsion is accompanied by increase of the oxygen partial pressure in the hypoxic tissues. Based on that result, they revealed availability of the combined use of PFC emulsion and chemical therapy against the hypoxic tissues under the condition that a peripheral oxygen partial pressure is kept at 300 mmHg and above. However, because of low oxygen affinity of the PFC emulsion, there remains such a problem that the PFC emulsion has to be used in a hyperbaric oxygen atmosphere, for example, in high-pressure tent.

On the other hand, Shorr et al. verified oxygenation of hypoxic tumor tissues and promotion effects on radiotherapy by administration of modified hemoglobin (polyethylene glycol (PEG)-modified hemoglobin (PEG-Hb) with a molecular weight of 128 kDa) (R. Linberg, C. D. Conover, K. L. Shum, R. G. S. Shorr, in vivo, 12, 167-174 (1998)). Four kinds of tumors, i.e., bone cancer, prostatic cancer, lung cancer and glioma were used as objects for investigation to determine changes of the oxygen concentration in the tumors by administration of various hemoglobin preparations. It was revealed that the PEG-Hb after a lapse of 2 hours from the administration maximized the increase of the oxygen concentration of hypoxic tumor tissues (4-7 Torr). Based on this fact, PEG-Hb was administered to rats transplanted with different radiosensitive cancers, which were then subjected to radiation of γ-rays to sequentially measure the sizes of the cancers. As a result, it was revealed that all the tumors are reduced in size. From these results, it was proved that PEG-Hb is effective for oxygenation of the hypoxic tumor tissues and for radiotherapy. However, it has generally been known that hemoglobin products are easy to leak out of the vascular endothelium, and trap vascular relaxing factors present in the immediate vicinities of the plain muscles, so that they induce vasoconstriction, and causes rapid enhancement of the blood pressure.

As is obvious, the oxygen infusions suitable for transporting molecular oxygen to the tumor tissues with small vessel diameters are those comprising molecules with particle size as small as possible. In other words, it is believed that the hypoxic region in the tumor tissues can be improved more effectively by an artificial oxygen carrier, which is small in molecules but has physicochemical features and molecular sizes that are hard to leak out from the vascular endothelium or kidney. However, it has been desired to fulfill design and synthesis of an oxygen infusion that meets these requirements, technical improvement in utilization and use.

Accordingly, the present invention has been made to overcome the aforesaid problems and is intended to provide an oxygen infusion with a high-safety for effectively increasing oxygen pressures in intratumoral hypoxic regions by administration to a site near the tumor tissues.

### Disclosure of the invention

The present inventors have studied on an artificial oxygen carrier compound that has a diameter smaller than that of conventional ones and a property to hardly leak out of the vascular endothelium or kidney, and on search, use and administration of an oxygen infusion containing said compound dispersed at a high concentration. As a result, the present inventors have found that a porphyrin metal complex that is an active center of oxygen coordination may be combined with serum albumin to form clathrates or inclusion complexes in which molecules of the porphyrin metal complex is enclosed within the crystal structure of serum albumin, and that the resultant porphyrin metal complex-albumin clathrate compounds can be used as preparations capable of providing oxygen to a hypoxic region in tumor tissues at a high efficiency. The present invention has been achieved by these findings.

According to the present invention, there is provided an oxygen infusion for increasing oxygen concentrations in tumor tissues in living bodies, said oxygen infusion comprising a dispersion of an albumin clathrate compound enclosing a porphyrin metal complex, said albumin clathrate compound being dispersed in a physiologically permissible aqueous media.

The present invention will be explained in detail hereinafter.

The present invention provides an oxygen infusion comprising an albumin compound enclosing a porphyrin metal complex. The oxygen infusion may comprise an albumin clathrate compound enclosing a porphyrin metal complex, dispersed in a physiologically permissible aqueous media, preferably, a physiological saline solution such as phosphate buffered saline.

The porphyrin metal complex used in the present invention is preferably a porphyrin metal complex represented by the general formula (I):
[Chem. 1] 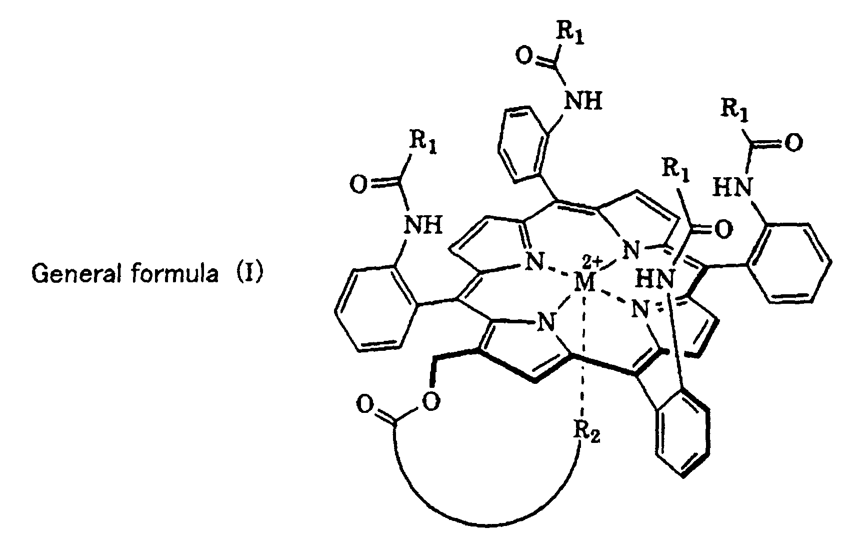 [wherein R₁ is a chain or alicyclic hydrocarbon group that may have one or more substituents,
R₂ is a basic axial ligand expressed by the formula (A):
[chem. 2] 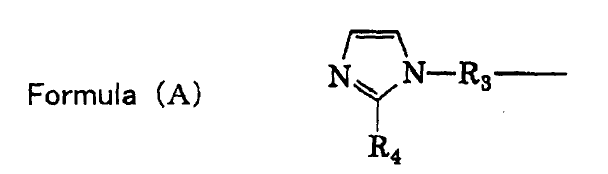 (where R₃ is alkylene, R₄ is a group that does not inhibit coordination of said basic axial ligand to a central transition metal ion M), or a basic axial ligand represented by the formula (B):
[Chem. 3] 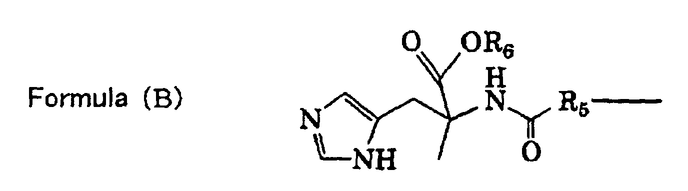 (where R₅ is alkylene, R₆ is alkyl); and
M is a transition metal ion of the 4th or 5th period of the periodic table of elements], and/or a porphyrin metal complex represented by the general formula [II]:
[Chem. 4]: 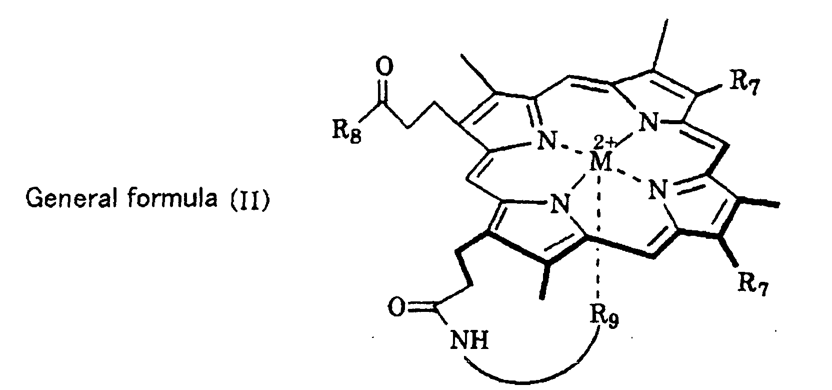 [wherein R₇ is hydrogen or a chain hydrocarbon group that may have one or more substituents,
R₈ is alkyloxy, alkylamino, or an amino acid or amino acid derivative residue,
R₉ is a basic axial ligand represented by the formula [C]:
[Chem. 5] 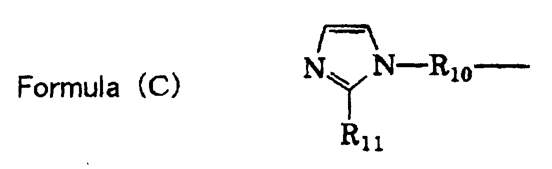 (where R₁₀ is alkylene, R₁₁ is a group that does not inhibit coordination of said basic axial ligand to a central transition metal ion M), or a basic axial ligand represented by the formula (D):
[Chem. 6] 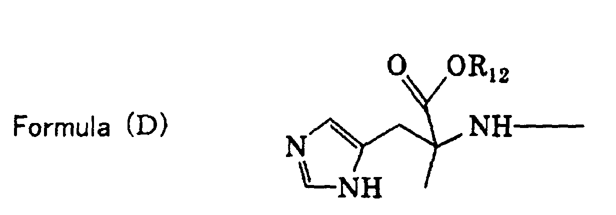 (where R₁₂ is alkyl), and
M is a transition metal ion of the 4th or 5th period of the periodic table of elements].

These porphyrin metal complexes constitute an active center for oxygen coordination.

In the porphyrin metal complexes of the general formula (I), it is preferred that R₁ is a C₁-C₁₉ chain hydrocarbon group having dimethyl groups at the first position, or a C₃-C₁₉ alicyclic hydrocarbon group having a substituent at the first position. Examples of the latter alicyclic hydrocarbon group include 1-substituted cyclopropyl, 1-substituted cyclopentyl, 1-substituted cyclohexyl, 1-methyl-2-cyclohexenyl, 2-substituted norbornyl and 1-substituted cycloadamantyl. Here, each substituent of the above groups may be methyl, C₁-C₁₈ alkylamide, C₁-C₁₈ alkanoyloxy, or C₁-C₁₈ alkoxy.

It is preferred that R₃ is C₁-C₁₀ alkylene.

It is preferred that R₄ is hydrogen, methyl, ethyl or propyl.

It is preferred that R₅ is C₁-C₁₀ alkylene.

It is preferred that R₆ is C₁-C₁₈ alkyl.

In the porphyrin metal complexes of the general formula (II), it is preferred that R₇ is hydrogen, vinyl, ethyl or methoxy.

Preferably, R₈ is C₁-C₁₈ alkyloxy, C₁-C₁₈ alkylamino, or an amino acid or its derivative residue. Preferably, the amino acid derivative is an amino acid-O-C₁-C₁₈ alkyl ester.

Preferably, R₁₀ is C₁-C₁₀ alkylene.

Preferably, R₁₁ is hydrogen, methyl, ethyl or propyl.

Preferably, R₁₂ is C₁-C₁₈ alkyl.

In both the general formulas (I) and (II), it is preferred that M is Fe or Co.

The porphyrin metal complexes of the general formula (I) are disclosed, for example, in JP H06-271577(A), and T. Komatsu et al., Chem. Lett., 2001, 668-669 (2001).

The porphyrin metal complexes of the general formula (II) are disclosed, for example, in T. G. Traylor et al., J. Am. Chem. Soc., 101, 6716-6731 (1979), JP S58-10388 (A) and JP S60-17326 (A), except for those in which R8 is alkylamino. Synthesis of the porphyrin metal complexes of the general formula (II) in which R8 is alkylamino are disclosed in examples mentioned below.

As the albumin compound enclosing the porphyrin metal complex, there may be used serum albumins such as human serum albumin, genetically-modified human serum albumin, bovine serum albumin and the like. As the albumin compounds, it is also possible to use albumins in the form of a multimer. In particular, it is preferred to use a dimeric form of albumin. The use of dimeric albumins makes it possible to prevent the clathrate compound from leaking out of the circulatory system.

For the production of oxygen infusions comprising an albumin clathrate compound enclosing a porphyrin metal complex therein, there may be used such a method as disclosed in JP S08-301873 (A), E. Tsuchida et al., Bioconjugate Chem., 10, 797-802 (1999), or Bioconjugate Chem., 11, 46-50 (2000). It is desirable that the oxygen infusion of the present invention generally contains the albumin compound at a concentration of 1 to 30 wt%, preferably, 5 to 25 wt%. The bonding number of the porphyrin metal complex per one molecule of the albumin compound is 1 to 8 (mol/mol), so that the concentration of the porphyrin metal complex ranges from 0.15 to 36 mM. An appropriate dosage of the oxygen infusion of the present invention is 40 mL/kg body weight or below.

The oxygen infusion of the present invention has the following favorable properties required for oxidization of tumor tissues: (i) Since the serum albumin that occupies about 60 % of plasma proteins is used as a carrier of the porphyrin metal complex that is an active center for oxygen coordination, the oxygen infusion of the present invention is very high in safety and biocompatibility at the time of intravascular administration; (ii) Since the molecular size is as small as 8 X 3 nm, the oxygen infusion can pass through small capillary vessels in the tumor tissues, to which the red blood cells (8 µm) can not reach; and (iii) the oxygen infusion has a low isoelectric point (pI), so that it does not leak out of the kidney or vascular endothelium.

Further, the oxygen affinity can be adjusted by controlling a three-dimensional structure of the porphyrin metal complex, thus making it possible to transport oxygen at a high efficiency according to the oxygen partial pressure in the affected part.

The oxygen infusion of the present invention makes it possible to increase the oxygen partial pressure in hypoxic regions of the tumor tissues by administrating it to mammalian living bodies with tumor tissues.

The oxygen infusion of the present invention can be administered to the living bodies by intra-arterial injection, intravenous injection, local administration, systemic administration or any other administrating means. Of course, the metal in the center of the porphyrin metal complex should be oxygenated before administration.

The following is a detained description of examples of a method for increasing oxygen partial pressures in hypoxic regions of tumor tissues, which is achieved by administrating the oxygen infusion of the present invention to mammals having tumor tissues.

Animals with cancer were prepared by transplanting tumor cells in desired sites of laboratory animals such as, for example, rats, hamsters, rabbits or beagles. The following is an explanation on transplantation of tumor cells into right femurs of rats.

Rats were bred for several days until tumors develop and then subjected to experiments under anesthesia (e.g., using Nembutal, diethyl ether or halothane anesthetic gas). Rats were intubated through the cervical trachea and ventilated under positive pressure by a respirator. A polyethylene catheter was inserted into a left carotid artery so that a distal end of the catheter is located at a position short of a bifurcation of the common iliac artery, and then backward cannulation was carried out to provide an administration rout of sample in the descending aorta.

The oxygen partial pressure in the tumor tissues may be determined by intravenously injecting a phosphorescent probe (palladium coproporphyrin (PdPor)), irradiating light, and monitoring a phosphorescence quenching time with an oxygen partial pressure monitor. PdPor is intravenously injected through a tail vein at 5-20 minutes before administration of the sample. The femur is incised by 20 mm to expose both normal muscles and the tumor, and the probe is arranged just above the tumor. The measurement is continued while preventing the tumor surfaces from being dried by wetting the tumor surfaces with a warm physiological saline solution of 37 °C. The oxygen partial pressure in the tumor site of the right leg was measured at 5 to 30 points in air or an atmosphere containing 99 % oxygen with a device for measurement of an oxygen partial pressure (e.g., OxySpot (Trademark) made by Medical System Corp.), and served as controls. Then, the oxygen saturated samples (1-20 mL/kg) were administered by intra-arterial injection for 1 to 20 minutes under a constant pressure with a syringe pump. Simultaneously with the initiation of intra-arterial injection, measurements were made on sequential changes of the oxygen partial pressure at both tumor site and normal site of the right leg. After completion of measurements, an incision is made at the abdominal area to confirm that the catheter for sample intra-arterial injection is located just before the bifurcation of the common iliac artery, and then measurements are made on size of the tumor.

In that case, there was observed no change in the oxygen partial pressure (PO2) in the tumor tissue even when the oxygen-saturated physiological saline, human serum albumin, genetically-modified human serum albumin, bovine serum albumin and albumin dimer were respectively intra-arterially injected. In contrast therewith, the administration of the oxygen infusion of the present invention containing porphyrin metal complex-albumin clathrate compound causes significant increase in the oxygen partial pressure in the tumor tissue. It is considered that the porphyrin metal complex-albumin clathrate compound of the present invention has a small particle size as mentioned above, and thus it can path through irregular blood vessels in the tumor tissue easily as compared with red blood cells, which in turn makes it possible to effectively increase the oxygen partial pressure in the tumor tissues.

### Brief Description of Drawings

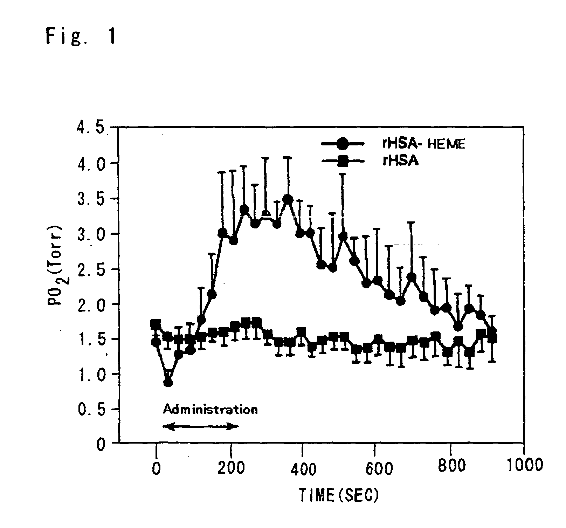

Fig. 1 is a graph illustrating changes of the oxygen partial pressure in tumor tissues resulting from administration of the oxygen infusion of the present invention.

### Best Mode for Carrying Out the Invention

The present invention will be explained below by examples but is never limited thereto.

### Synthetic Example A: Synthesis of 8, 13-bisvinyl-2-methyl aminocarbonylethyl-18-(3-(1-imidazolyl)propylamino)carbonyl ehtyl-3, 7, 12, 17 tetramethylporphyrin iron complex

### (I) Synthesis of 8, 13-bisvinyl-2-methyl aminocarbonyl ethyl-18-(3-(1-imidazolyl) propylamino)carbonyl ehtyl-3, 7, 12, 17-tetramethyl porphyrin

Protoporphyrin IX (0.1 g, 0.18 mmol), distilled pyridine (10 ml) and triethylamine (51 µL, 0.45 mmol) were charged into a three neck recovery flask of 100 ml and stirred for 10 minutes. The resultant mixture was added with (benzotriazolyloxy) tris (dimethylamino) phosphonium hexafluorophosphate (124 mg, 0.45 mmol), stirred for 10 minutes, added with 1-(3-aminopropyl) imidazole (15 µL, 0.14 mmol) and then stirred for 4 hours under the light shielding condition. The resultant reaction mixture was added with 5 mL of tetrahydrofuran solution of methylamine, and stirred for 4 hours. After removing pyridine under reduced pressure by a vacuum pump, the reaction products were fractionated and refined with silica gel columns (chloroform/methanol/tryethylamine=8/1/1). The resultant fraction was vacuum dried. There was obtained a red solid substance, 24 mg (yield 19 %) of 8, 13-bisvinyl-2-methyl aminocarbonylethyl-18-(3-(1-imidazolyl)propylamino) carbonylehtyl-3, 7, 12, 17-tetramethylporphyrin.
Rf: 0.6 (chloroform/methanol=3/1)
IR(cm⁻¹) : νc=O (amido) : 1631
UV-vis/λmax (nm) (CHCl3) : 631; 579; 542; 508; 409
1H-NMR (δ (ppm)) (CDCl3) : -4.0 (s, 2H, inner); 1.8-2.4 (m, 4H, -C₂H₄-Im); 2.7 (m, 4H, -CH₂-COO-, -CH₂-NH-); 3.5-3.7 (m, 15H, Por-CH₃, CONHCH₃); 4.2 (s, 4H, Por-CH₂-); 5.8 (s, 1H, Im); 6.1-6.4 (q, 4H, CH₂=CH-); 6.6 (d, 1H, Im); 8.2 (m, 2H, CH₂=CH-); 9.5-10.0 (q, 4H, meso)
FAB-MAS[m/Z] : 683.

### (II) Synthesis of iron complex

A three neck recovery flask of 50 ml was charged with 5 ml of a dimethylformamide solution of the porphyrin compound (23.9 mg, 34.6 µmol) obtained in the above process (I), and then deoxygenated with nitrogen for 20 minutes. Iron chloride tetrahydrate (68.8 mg, 346 µmol) was quickly added to that solution, and the resultant mixture was stirred under the nitrogen atmosphere for 3 hours at 70 °C. The completion of reactions was confirmed by adding one drop of hydrochloric acid to a diluted solution of the reaction mixture in chloroform and observing no peaks (at about 450 nm) derived from dication in the UV-visible spectrum. The solvent was removed under reduced pressure by a vacuum pump, and the reaction products were fractionated and refined with silica gel columns (chloroform/methanol/tryethylamine=8/1/1). By vacuum drying, there was obtained 7.6 mg (yield: 29 %) of a brown solid substance.
Rf: 0.1 (chloroform/methanol/tryethylamine=8/1/1)
IR(cm⁻¹) : νc=O (amido):1649
UV-vis/λmax (nm) (CHCl3) : 583; 531; 406
FAB-MAS[m/Z] : 736.

### Synthetic Example B: Synthesis of 8, 13-bisvinyl-2-dodecyl aminocarbonylethyl-18-(methyl-O-histidine)carbonylethyl-3,7,12,17-tetramethyl porphyrin iron complex

There was obtained 8, 13-bisvinyl-2-dodecyl amino carbonylethyl-18-(methyl-O-histidine) carbonylethyl-3, 7, 12, 17-tetramethylporphyriniron with a yield of 25% in the same manner as the process (I) of synthetic Example A except for that histidine-O-methyl ester was used instead of 1-(3-aminopropyl)imidazole, and that dodecylamine was used instead of methylamine.
Rf: 0.4 (chloroform/methanol = 15/1)
IR(cm⁻¹) : νc=O (amido):1640
UV-vis/λmax (nm) (CHCl3) : 631; 575; 540; 409
1H-NMR (δ(ppm)) (CDCl3) : -4.0 (s, 2H, inner); 0.8 (s, 3H, -(CH₂)₁₀CH₃); 1.2-1.8 (m, 20H, -CH₂-); 1.9 (t, 4H, -CH₂(C=O)NH-); 3.2 (s, 2H, -Im-CH₂-); 3.3 (t, 2H, -(C=O)NHCH₂-); 3.5-3.7 (m, 12H, Por-CH3); 3.7 (m, 3H, His-OMe); 4.2 (s, 4H, Por-CH₂-); 4.8 (s, 1H, His-CH₂CH-); 6.1-6.4 (q, 4H, CH₂=CH-); 6.8 (s, 1H, Im); 7.6 (s, 1H, Im); 8.2 (m, 2H, CH₂=CH-); 9.5-10.0 (q, 4H, meso)
FAB-MAS [m/Z] : 881

Using the resultant porphyrin, its iron complex was prepared in the same manner as the process (II) of synthetic example A.

### [Example 1]

### Preparation of the oxygen infusion of the present invention

A separable flask (2 L) was charged with 10 mL (2.5 mg, 37.5 µmol) of human serum albumin (25 wt%) and 1 L of phosphate buffered saline (pH 8.1), and then provided with a dropping funnel of 500 mL. Separately, a recovery flask (300 mL) was charged with 250 mL of an ethanol solution of 2-8-(2-methyl-1-imidazolyl) octanoyloxymethyl-5, 10, 15, 20-tetrakis- (α,α,α,α-o-pivaloylamidophenyl) porphyrin iron (II) complex (hereinafter referred to as "FepivP (Im)", 390 mg, 300 µmol), and connected to the above separable flask through a Teflon (Trademark) tube. The tube was kept so as not to come into contact with the liquid surface. Carbon monoxide was bubbled in the recovery flask containing the ethanol solution of FepivP (Im), and the exhaust gas thereof was allowed to flow to the albumin solution. Simultaneously therewith, the bubbling was carried out so as not to foam the albumin solution. The bubbling and the exhaust gas flow were carried out for about 60 minutes. In the carbon monoxide atmosphere, the ethanol solution of FepivP (Im) was added with 250 µL of an aqueous solution of ascorbic acid (0.6 M) and stirred for 5 minutes. In this manner, Heme is reduced to form a carbon monoxide complex and color of the solution was changed to magenta.

The resultant ethanol solution of the carbon monoxide FepivP (Im) complex was transferred to the dropping funnel mounted on the separable flask, and slowly dropped into the albumin solution. After completing the dropping, the solution was stirred for 30 minutes.

The following procedures were carried out under the light shielding conditions using aluminum foils. Using a closed-circuit type ultrafiltration equipment (Pellicon 2 MINI HOLDER (Trade name), Cat. NO. XX42PMINI) provided with a ultrafiltration membrane having a filtration area of 0.1 m² and a molecular weight cut off of 10 kDa, P2B010A01 (Trade name of MILIPORE), 1.25 L of the 20 % ethanol solution of the resultant albumin-Heme was filtered. At the time 50 mL of a filtrate was filtered out, 50 mL of a phosphate buffer (1 mM, pH 7.3) was added. This procedure was repeated until 10 L (1.25 X 8 L) of the phosphate buffer solution was filtered out. Then, the albumin-Heme solution was concentrated to 100 mL and collected into a container.

The resultant concentrated solution (about 200 mL) was filtered with a filter (DISMIC (Trade name), 0.45 µm), and the filtrate was concentrated by an ultrafiltration unit (UHP-76K, ADVANTEC (Trademark)) to obtain a concentrated solution of about 50 mL.

The resultant concentrated solution was added with a 20 wt% solution of sodium chloride so that a concentration of sodium chloride becomes 140 mM.

Using a pH meter and a salt meter, measurements were made on pH and a Na⁺ concentration of the albumin-Heme solution. The albumin concentration was determined by a bromocresol green (BCG) test, while the concentration of FepivP (Im) was determined by an ICP (inductively coupled plasma) emission spectrometry.

A recovery flask of 100 mL was charged with the albumin-Heme (carbon monoxide complex) solution (20 mL) and attached to a rotary evaporator. While cooling the recovery flask with an ice water bath, the recovery flask was rotated and subjected to oxygen flowing through an upper stopcock of a cooling tube for 20 minutes. Then, a halogen lamp (500 w) was fixed to a position spaced by 15 to 20 cm above the rotating recovery flask, and turned on to irradiate the albumin-Heme solution for 10 minutes. Formation of an oxygen complex was confirmed from an absorbance and λmax of ultraviolet visible spectrum of the albumin-Heme solution. A molar extinction coefficient (ε426) of the obtained oxygen complex was about 1.16×10⁵ M⁻¹cm⁻¹. Measurements of oxygen partial pressures in healthy cells and tumor cells

As experimental animals, there were used Donryu rats (Crj-Donryu; Nippon Charles River, male, weight: about 200 g, 6 week old) that were bred by a biological clean system under free-feeding of pellets and water. The transplanted tumor cells, Ascites hepatoma LY80, were developed by intra-abdominal passage transplantation. For preparation of rats developed by a cancer, there were used tumor cells grown for 7 days after transplantation. At 8 days before transplantation, incision was made in the right femur of the rat, and 5 X 10⁶ cells were transplanted just beneath the muscle of thigh with syringes of 27 G and 1 mL. The partial pressure of oxygen in the tumor tissues was determined from a phosphorescence quenching time that was monitored with an oxygen partial pressure measurement system (Oxyspot Photomeric Oxygen Measurement System (OxySpot, Medical System Corp.) after light irradiation following intravenous injection of palladium coproporphyrin (PdPor). PdPor was intravenously injected by an indwelling needle 24 G through the tail vein at 15 minutes before administration of the sample. A small incision of 20 mm was made in the femur to expose both normal muscle and the tumor, and a measuring probe was located just above the tumor at a distance of 5 mm. The measurements were carried out while preventing the tumor surfaces from being dried by rinsing the tumor surface with warm physiological saline of 37 °C.

The rats were anesthetized by inhalation with ether, intubated with 14G-Angiocath (Trademark) via their cervical trachea, and ventilated under positive pressure (80 times/min) by an artificial respirator (Respirator Model SN 480-7 made by Shinano MFG., CO. Ltd), while feeding a gas of 1 % halothane anesthetic gas (FiO2, 1 % halothane Fluothane) mixed with air or oxygen through a small animal anesthetizer. A polyethylene catheter (SP10, single lumen, inside diameter: 0.28 mm, outer diameter: 0.61 mm) was inserted into a left carotid artery and located at a distance of 1 cm short of a common iliac artery bifurcation(about 9.5 cm), and then reverse cannulation was carried out to keep a sample administration line in the descending aorta.

After peeling away a skin of tumor cells of the right leg, PdPor (0.24 cc (10 mg/mL, 0.9 mL/kg)) was injected through the tail vein of caudal portion of the rat. The partial pressures of oxygen were measured at 20 points of tumor sites of the right leg in air and an atmosphere containing 99 % oxygen using OxySpot (Trademark), and served as controls. Then, oxygen-saturated samples (10 mL/kg) were administered by intra-arterial injection for duration of 4 minutes (2.5 mL/kg/min) under a constant pressure with a syringe pump (FP-W-100, Matys, ToyoSangyo Ltd.). Simultaneously with the initiation of intra-arterial injection, measurements were made on the partial pressures of oxygen at both tumor sites and normal sites (at 5 points, for a duration of 15 minutes) to determine sequential changes of the oxygen partial pressure. After the measurements, an incision was made in the abdomen to confirm a fact that the sample intra-arterial injection catheter is located at a position short of the bifurcation of common iliac artery, and a size of the tumor was measured.

The partial pressures of oxygen (PO2) at the normal sites and tumor sites of the right leg under the 99 % oxygen atmosphere were 14-16 Torr and 1.4-1.7 Torr, respectively, as shown in Table 1. The partial pressures at the tumor sites are considerably low as compared with those at the normal sites. Thus, it was confirmed that the partial pressure is not increased only by increase in the oxygen concentration in breathing of the rat.

The primary values of various parameters for the rHSA-Heme treated group (4 rats) and rHSA treated group (4 rats) are shown in Table 1.

**[TABLE 1]**

| | rHSA-Heme treated group | rHSA treated group |
|---|---|---|
| weight(g) | 213±4.2 | 203±6.3 |
| tumor size(mm) | 16.5×14.0 | 19.0×14.0 |
| PO₂(Torr) (tumor cells) | 1.4±0.2 | 1.7±0.2 |
| PO₂ (Torr) (tumor cells) | 15.7±2.3 | 14.8±2.8 |
| Blood gas parameter: | | |
| pH | 7.41±0.04 | 7.42±0.04 |
| PO₂(Torr) | 394±73 | 402±60 |
| PCO₂ (Torr) | 34.6±4.2 | 33.0±2.8 |

Next, oxygen-saturated, genetically modified human serum albumin (rHSA) was administered by intra-arterial injection, but there was no change in the oxygen partial pressure (PO₂). In contrast therewith, when the oxygen infusion product of the present invention prepared as above was administered, the oxygen partial pressure in the tumor tissues was increased up to 3.5 Torr. These results are shown in Fig. 1. The oxygen partial pressure after administration of the oxygen infusion product of the present invention is 2.5 time of that before administration. It is considered that the porphyrin metal complex-clathrate albumin compound of the present invention can effectively increases the oxygen partial pressure in the tumor tissues since the compound can pass easily through the irregular blood vessels in the tumor tissues because of its small molecular size, as compared with the red blood cells.

### [Example 2]

Effects of oxygen supply to the hypoxic region in the tumor tissues were measured in the same manner as Example 1, except for that 2-8-(1-imidazolyl) octanoyloxy methyl-5, 10, 15, 20-tetrakis-(α,α,α,α-o-(1-methyl cyclohexanoyl) aminophenyl)porphyrin iron(II) complex was used instead of FepivP (Im) in Example 1. The oxygen partial pressure in the tumor tissues was increased up to about 7.0 Torr. Thus, it was demonstrated that the effect of supplying oxygen to the low oxygen tumor tissues that results from the administration of albumin-Heme, which is an artificial oxygen carrier.

### [Example 3]

Effects of oxygen supply to the hypoxic region in the tumor tissues were measured in the same manner as Example 1 except for taht 8, 13-bisvinyl-2-methoxycarbonyl ethyl-18-(3-(1-imidazolyl) propylamino) carbonylehtyl-3, 7, 12, 17-tetramethyl porphyrin iron(II) complex was used instead of FepivP (Im) in Example 1. The oxygen partial pressure in the tumor tissues was increased up to about 1.6 Torr. Thus, it was demonstrated that the effect of supplying oxygen to the low oxygen tumor tissues that results from the administration of albumin-Heme which is an artificial oxygen carrier.

### Industrial Applicability

As mentioned above, according to the present invention there is provided a highly safe oxygen infusion product for effectively increasing an oxygen partial pressure in hypoxic region of tumor tissues when administered to sites near tumor tissues.

## Claims

1. An oxygen infusion for increasing an oxygen concentration in tumor tissues in living bodies, said oxygen infusion comprising a dispersion of an albumin clathrate compound including porphyrin metal complex, dispersed in a physiologically permissible aqueous media.

2. The oxygen infusion according to claim 1, wherein said porphyrin metal complex is a porphyrin metal complex represented by the general formula (I):
[Chem. 7] 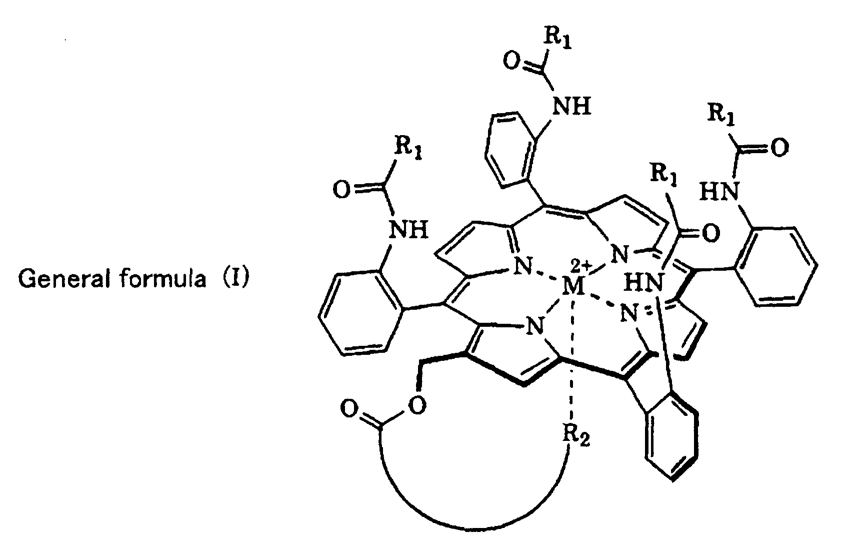 [wherein R1 is a chain or alicyclic hydrocarbon group that may have one or more substituents,
R2 is a basic axial ligand expressed by the formula (A) :
[chem. 8] 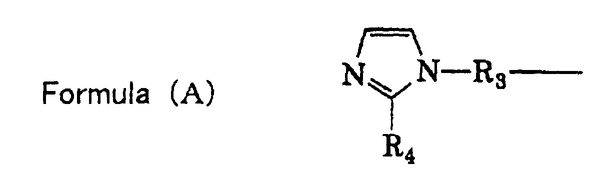 (where R3 is alkylene, R4 is a group that does not inhibit coordination of said basic axial ligand to a central transition metal ion M), or a basic axial ligand represented by the formula (B):
[Chem. 9] 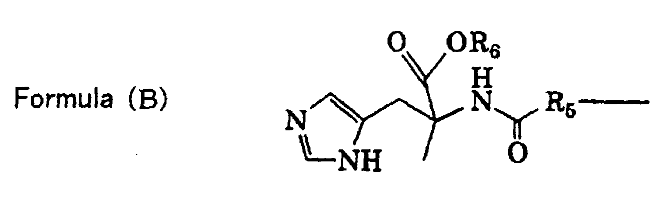 (where R5 is alkylene, R6 is alkyl); and
M is a transition metal ion of the 4th or 5th period of the periodic table of elements], and/or a porphyrin metal complex represented by the general formula [II]:
[Chem. 10] 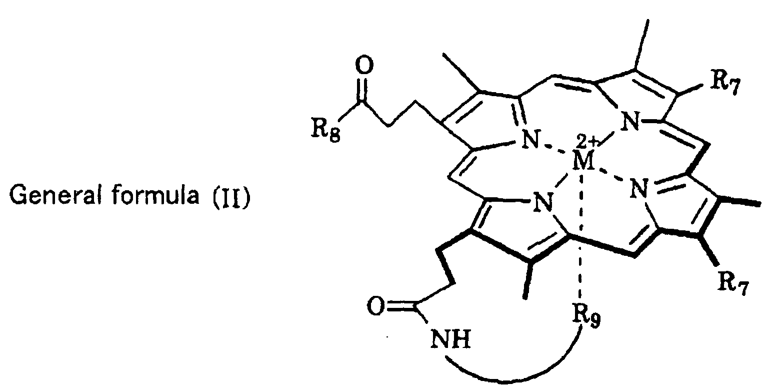 [wherein R7 is a chain or alicyclic hydrocarbon group that may have one or more substituents,
R8 is alkyloxy, alkylamino, amino or an amino acid derivative residue,
R9 is an basic axial ligand represented by the formula [C] :
[Chem. 11] 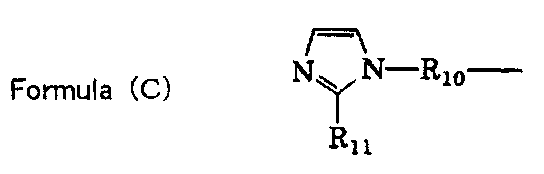 (where R10 is alkylene, R11 is a group that does not inhibit coordination of said basic axial ligand to a central transition metal ion M), or an basic axial ligand represented by the formula (D):
[Chem. 12] 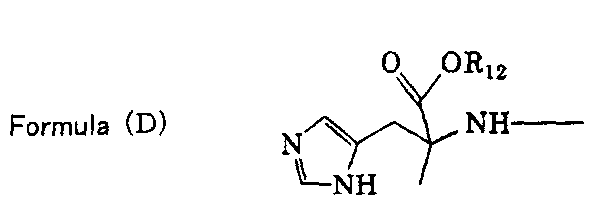 (where R12 is alkyl), and
M is a transition metal ion of the 4th or 5th period of the periodic table of elements].

3. The oxygen infusion according to claim 2, wherein said porphyrin metal complex is a porphyrin metal complex of the general formula (I), in which R₁ is C₁-C₁₉ chain hydrocarbon group having dimethyl groups at the first position or C₃-C₁₉ alicyclic hydrocarbon having a substituent at the first position, R₃ is C₁-C₁₀ alkylene, R₄ is hydrogen, methyl, ethyl or propyl, R₅ is C₁-C₁₀ alkylene, R₆ is C₁-C₁₈ alkyl, M is Fe or Co.

4. The oxygen infusion according to claim 2, wherein said porphyrin metal complex is a porphyrin metal complex of the general formula (II), in which R₇ is hydrogen, vinyl, ethyl or methoxy; R₈ is C₁-C₁₈ alkyloxy, C₁-C₁₈ alkylamino, amino acid or a derivative residue thereof; R₁₀ is C₁-C₁₀ alkylene; R11 is hydrogen, methyl, ethyl or propyl; R₁₂ is C₁-C₁₈ alkyl; and M is Fe or Co.
